# FASCICULE DE BREVET EUROPEEN

(11) **EP 0 500 423 B1**
(45) Date de publication et mention de la délivrance du brevet: **24.01.1996**
(21) Numéro de dépôt: 92400379.1
(22) Date de dépôt: 13.02.1992
(51) Int. Cl.: A61K 7/50, A61K 7/06, A61K 7/48

(54) **Composition de lavage à base d'huile de synthèse et procédé de mise en oeuvre**
Syntheseöle enthaltende Reinigungsmittel und Verfahren zur Ausführung
Cleansing composition containing synthetic oil and method for carrying it out

(30) Priorité: 19.02.1991 FR 9101954
(43) Date de publication de la demande: 26.08.1992
(73) Titulaire: L'OREAL, F-75008 Paris (FR)
(72) Inventeur: Dubief, Claude, F-78150 Le Chesnay (FR); Cauwet, Danièle, F-75011 Paris (FR)
(74) Mandataire: Casalonga, Axel

(56) Documents cités:
- EP-A- 0 217 250
- BE-A- 1 000 757
- FR-A- 2 596 061
- GB-A- 2 164 658

## Description

L'invention est relative à des compositions de lavage et de conditionnement des matières kératiniques, en particulier des cheveux et/ou de la peau, et comportant dans un milieu aqueux, au moins une huile synthétique en présence d'agents de mise en suspension de cette huile et d'agents tensio-actifs détergents, ainsi qu'aux procédés de lavage mettant en oeuvre ces compositions.

Les compositions de lavage des matières kératiniques, notamment les shampooings, sont bien connues dans l'état de la technique.

On a déjà proposé d'utiliser des huiles dans de telles compositions, notamment dans le traitement des cheveux secs en vue de leur conférer de la douceur et de la brillance.

Du fait du caractère insoluble de ces huiles synthétiques, dans les milieux aqueux habituellement utilisés dans les compositions de lavage telles que les shampooings, on cherche à maintenir ces huiles sous forme dispersée tout en conservant les propriétés détergentes et moussantes de la composition, sans faire chuter la viscosité. Ces compositions doivent également conférer aux cheveux des propriétés de douceur, de brillance et de démêlage recherchées.

EP-A-0 217 250 divulgue une composition tensio-active et moussante pouvant contenir, en dehors d'agents de mise en émulsion et d'agents tensio-actifs, des huiles faiblement volatiles parmi lesquels sont cités les hydrocarbures ramifiés tels que par exemple les isoparaffines ayant 16 à 30 atomes de carbone.

FR-A-2 596 061 décrit une composition pour le lavage des mains comprenant principalement un mélange d'eau et de solvant de type aliphatique isoparaffinique, ainsi que des agents tensio-actifs détergents et des agents moussants.

La demanderesse a découvert que, de façon surprenante, il était possible de préparer des compositions de lavage, particulièrement stables dans le temps et possédant de bonnes propriétés de détergence, tout en conférant aux cheveux de la brillance, de la douceur, une facilité de peignage sans toucher collant, en utilisant dans ces compositions, en plus des agents tensio-actifs détergents, des agents particuliers de mise en suspension de ces huiles synthétiques.

On constate en plus que ces compositions ne présentent pas l'inconvénient d'alourdir la chevelure lorsqu'elles sont utilisées de façon répétée.

Les compositions conformes à l'invention présentent, par ailleurs, l'avantage de pouvoir être réalisées sans difficulté à l'aide des procédés et des matériels classiquement utilisés.

L'invention a donc pour objet une composition lavante contenant au moins une huile de synthèse, au moins un agent de mise en suspension de cette huile et au moins un agent tensio-actif détergent.

Un autre objet de l'invention est constitué par l'utilisation des agents de suspension définis ci-après comme agents de suspension d'huiles synthétiques dans un milieu aqueux contenant des tensioactifs détergents.

L'invention a également pour objet un procédé de lavage mettant en oeuvre les compositions définies ci-dessus.

D'autres objets de l'invention apparaîtront à la lecture de la description et des exemples qui suivent.

Les compositions de lavage conformes à l'invention, comprennent dans un milieu aqueux:
(A) au moins une huile synthétique choisie parmi :
   (i) les isoparaffines de structure : 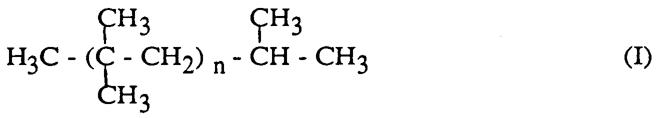 où n est compris entre 2 et 16 inclus;
   (ii) le mélange des isoparaffines de formule (I) avec des isoparaffines de formule (II) : 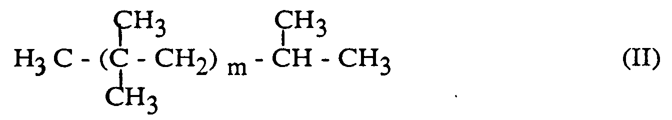 où m est égal ou supérieur à 18, et de préférence compris entre 18 et 40;
      la viscosité capillaire des huiles définies ci-dessus étant inférieure à 500 cps;
(B) un agent de mise en suspension de l'huile, choisi parmi les composés de formule:
   a)

      RX (III)

      dans laquelle R est un radical aliphatique à longue chaîne carbonée, éventuellement interrompue par un ou plusieurs atomes d'oxygène, et X est un reste acide carboxylique, sulfurique ou phosphorique ou un radical dérivé d'un acide carboxylique ou d'un amide; ces composés de formule (III) sont choisis parmi ceux dans lesquels :
      (i) R est un radical alkyle ou alcényle en C₁₁-C₂₁;
         et X est
         . un groupement COOA où A est un radical mono- ou polyhydroxyalkyle dérivé d'un polyol en C₂-C₃ ou un radical CH₂CH₂SO₃M;
         . un groupement CO(OCH₂CH₂)ₖOH où k a une valeur comprise entre 2 et 150;
         . un groupement 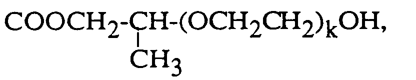 où k a une valeur comprise entre 2 et 150, les fonctions OH libres des groupements définis ci-dessus pouvant être estérifiées par un acide RCOOH où R est un alkyle ou alcényle en C₁₁-C₂₁;
         . un groupement CONR₁R₂ où R₁ et R₂ représentent hydrogène ou hydroxyalkyle en C₁-C₄, l'un au moins représentant hydroxyalkyle en C₁-C₄;
         . un groupement OSO₃M ou 1/3 PO₄ ³⁻ M₃ où M représente un métal alcalin, ammonium ou un reste d'alcanolamine en C₁-C₄;
      (ii) R désigne un radical R₃O(C₂H₄O)_{ℓ}CH₂ et X désigne un groupement COOM où M a la signification indiquée ci-dessus, R₃ désignant un radical alkyle en C₁₂-C₁₄ et ℓ un nombre entier ou décimal compris entre 2,5 et 10, ou bien R₃ désigne oléyle et ℓ varie de 2 à 9 ou encore R₃ désigne alkyle en (C₈-C₉) phényle et ℓ varie de 4 à 8, ou les dérivés dans lesquels R₃ désigne un groupement alkyle (C₁₂-C₁₆) et X un groupement CONR₁R₂, dans lequel R₁ et R₂ ont la même signification que celle indiquée ci-dessus et ℓ a une valeur de 1 à 3 inclus;
   b) des oxydes d'amines de formule : 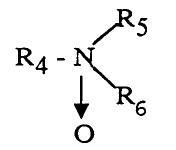 dans laquelle R₄ désigne un groupement alkyle en C₁₆-C₂₂, et R₅ et R₆, identiques ou différents, représentent un groupement alkyle en C₁-C₄ ou hydroxyalkyle en C₁-C₄;
   c) les biopolysaccharides choisis parmi les gommes de xanthane et les scléroblucanes;
(C) et un agent tensio-actif détergent.

Parmi les huiles synthétiques répondant à la formule (I) définie ci-dessus, on peut citer celles dans lesquelles n est égal à 2, 3, 4 ou 16, et en particulier les produits vendus sous les dénominations PERMETHYL 99A, 101A, 102A ou 104A par la Société PRESPERSE INC ou le produit ARLAMOL HD vendu par la Société ICI, correspondant à la formule (I) dans laquelle n est égal à 3.

Parmi les huiles synthétiques de formule (II), on peut citer le produit vendu sous la dénomination commerciale de PERMETHYL 106A, répondant à la formule (II) dans laquelle m est égal à 38.

Parmi les agents de suspension de formule RX préférés, on peut citer :
- les esters d'acides gras en C₁₂-C₂₂ et de polyols en C₂-C₃ tels que les myristates, palmitates et stéarates d'éthylèneglycol, de propylèneblycol ou de glycérol et plus particulièrement le monostéarate ou le distéarate d'éthylèneglycol, le mono- ou distéarate ou tristéarate de glycérol;
- les alcanolamides d'acides gras en C₁₂-C₂₂ et plus particulièrement le diéthanolamide de coprah ou stéarique, le monoéthanolamide de coprah ou stéarique, le monoisopropanolamide de coprah ou stéarique;
- les alkyléthers d'alcanolamides tels que l'alkyl(C₁₃-C₁₅)oxydiéthoxyméthylmonoéthanolamide, comme par exemple le produit vendu sous la dénomination AMINOL A15 par la Société CHEM'Y;
- les acides gras en C₁₆-C₂₂ polyoxyéthylénés tels que les stéarates de polyéthylèneblycol, comportant entre 4 et 150 motifs éthoxy;
- les esters d'acides gras en C₁₆-C₂₂ et de propylèneglycol polyoxyéthylénés et estérifiés, pouvant comporter entre 4 et 100 motifs éthoxy;
- les acides d'éthers carboxyliques polyoxyéthylénés, répondant à la formule R₃O(C₂H₄O)_{ℓ}CH₂COOM (IV), parmi lesquels les produits vendus sous les dénominations AKYPO RLM 38, 25, 45 et 100, pour lesquels R₃ représente respectivement une chaîne alkyle en C₁₂-C₁₄ et ℓ désigne respectivement 3,8; 2,5; 4,5 et 10; le produit AKYPO RO 50 pour lequel R₃ désigne un radical oléyle et ℓ est égal à 5; des mélanges de ces composés et plus particulièrement un mélange du produit AKYPO RO 20 et RO 90 pour lesquels R₃ désigne oléyle et ℓ est égal respectivement à 2 et à 9; ou bien le mélange des produits AKYPO NP 40 et NP 70, dans lesquels R₃ désigne nonylphényle et ℓ est égal à 4 et 7; ou bien les produits AKYPO OP 40 et OP 80, dans lesquels R₃ représente octylphényle et ℓ est égal à 4 et 8, les produits AKYPO étant vendus par la Société CHEM'Y.

Les oxydes d'amines sont par exemple des oxydes d'alkyl(C₁₆-C₂₂) de diméthylamine, tel que l'oxyde de stéaryldiméthylamine.

Les biopolysaccharides utilisés sont des produits comportant dans leur structure des unités blucose, mannose, acide glucuronique ou galacturonique ou D-glucopyranose..

Parmi ces composés, on peut citer la gomme de xanthane obtenue par l'action de la bactérie XANTHOMONAS CAMPESTRI et les mutants ou variants de celle-ci ayant un poids moléculaire compris entre 1.000.000 et 50.000.000. Les gommes de xanthane ont une viscosité comprise entre 0,60 et 1,65 Pa.s pour une composition aqueuse contenant 1% de gomme de xanthane, mesurée au viscosimètre Brookfield, type LVT à 60 tours/minute. Ces gommes, qui sont des hétérobiopolysaccharides, comportent dans leur structure 3 monosaccharides différents qui sont le mannose, le glucose et l'acide glucuronique. Les produits particulièrement préférés sont ceux commercialisés sous la dénomination "KELTROL" par la Société KELCO, KELZAN S commercialisé par la Société KELCO, RHODOPOL 23 SC commercialisé par la Société RHONE-POULENC, RHODIGEL 23 vendu par la Société RHONE-POULENC, DEUTERON XG commercialisé par la Société SCHOENER GmbH, l'ACTIGUM CX9 commercialisé par la Société CECA/SATIA, les produits vendus par la Société KELCO sous les dénominations "KELZAN K3 B130, K8 B12".

D'autres biopolysaccharides utilisables coriformément à l'invention peuvent être choisis parmi le biopolymère PS 87 engendré par la bactérie BACILLUS-POLYMYXA qui comprend dans sa structure du glucose, du galactose, du mannose, du fucose et de l'acide glucuronique; ce biopolymère PS 87 est décrit dans la demande de brevet européen publiée n° 23397; le biopolymère S88 engendré par la souche PSEUDOMONAS ATCC 31554, qui comprend dans sa structure du rhamnose, du glucose, du mannose et de l'acide glucuronique; ce biopolymère est décrit dans le brevet britannique n° 2 058 106; le biopolymère S130 engendré par la souche ALCALIGENES ATCC 31555, qui comporte dans sa molécule du rhamnose, du glucose, du mannose et de l'acide glucuronique, ce biopolymère est décrit dans le brevet britannique n° 2 058 107; le biopolymère S139 engendré par la souche PSEUDOMONAS ATCC 31644, qui comprend dans sa molécule du rhamnose, du glucose, du mannose, du balactose et de l'acide galacturonique; ce biopolymère est décrit en particulier dans le brevet US n° 4 454 316; le biopolymère S198 engendré par la souche ALCALIGENES ATCC 31853, qui comprend dans sa molécule du rhamnose, du glucose, du mannose et de l'acide blucuronique; ce biopolymère est décrit dans la demande de brevet européenne 64 354.

Le biopolymère BM07 décrit dans la demande européenne EP 351 303, comportant dans sa molécule des motifs dérivés du glucose, du galactose et des acides pyruvique, succinique et acétique.

Le biopolymère AM-2 qui comporte dans sa molécule du glucose, du rhamnose, du mannose et de l'acide glucuronique, décrit dans la demande européenne 127 698 ou ceux décrits dans la demande européenne 79038 comportant dans leur molécule du glucose, du galactose, du mannose et de l'acide glucuronique.

Les scléroglucanes utilisés conformément à l'invention, sont des polysaccharides neutres d'origine microbienne, obtenus par fermentation aérobie d'un milieu glucosé par un champignon du type Sclerotium et présentant la structure d'un homopolymère de D-glucopyranose.

Les scléroglucanes répondent à la formule : 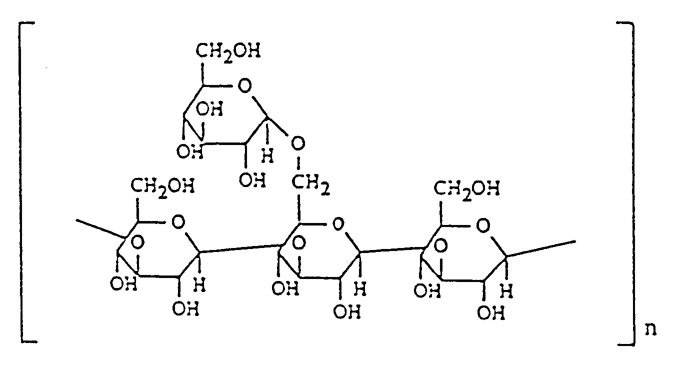
où n (degré de polymérisation) varie de 500 à 1600.

Les scléroglucanes utilisés conformément à l'invention, sont représentés par les produits vendus sous la dénomination ACTIGUM CS par la Société SANOFI BIO INDUSTRIES et en particulier ACTIGUM CS 11 et sous la dénomination AMIGEL par la Société ALBAN MULLER INTERNATIONAL. D'autres scléroglucanes tels que celui traité au glyoxal décrit dans la demande de brevet FR 2 633 940, peuvent également être utilisés.

Les agents tensio-actifs utilisés dans les compositions de lavage conformes à l'invention, sont choisis parmi les agents tensio-actifs anioniques, amphotères, zwittérioniques, non-ioniques ou leurs mélanges, ayant des propriétés détergentes.

Parmi les agents tensio-actifs anioniques, on peut citer les sels alcalins, les sels d'ammonium, les sels d'amines, les sels d'aminoalcools, les sels de magnésium des composés suivants : les alkyléthersulfates, alkylamidoéthersulfates, alkylarylpolyéther sulfates, monoglycérides sulfates; les alkylsulfonates, alkylamides sulfonates, alkylarylsulfonates, oléfines sulfonates, paraffines sulfonates; les alkylsulfosuccinates, les alkyléthersulfosuccinates, les alkylamides sulfosuccinates; les alkylsulfosuccinamates; les alkylsulfoacétates; les alkylétherphosphates; les acylsarcosinates, les N-acyltaurates. Le radical alkyle ou acyle de ces différents composés étant constitué par une chaîne carbonée comportant de 12 à 20 atomes de carbone.

Les alkylsulfates, les alkylphosphates dont le radical alkyle comporte 12 à 14 atomes de carbone.

Parmi les abents tensio-actifs anioniques, on peut également citer les sels d'acides gras tels que les sels des acides oléique, ricinoléique, palmitique, stéarique; les acides d'huile de copra ou d'huile de copra hydrogénée; les acyl lactylates, dont le radical acyle comporte de 8 à 20 atomes de carbone.

On peut également utiliser des agents tensio-actifs faiblement anioniques, tels que les acides éthers carboxyliques polyoxyalkylénés, en particulier ceux comportant 2 à 50 groupements oxyde d'éthylène.

Les agents tensio-actifs non-ioniques sont choisis parmi les alcools ou les α-diols ou les alkylphénols ou les acides gras polyéthoxylés, polypropoxylés ou polyglycérolés, à chaîne brasse comportant 8 à 18 atomes de carbone, le nombre de groupements oxyde d'éthylène ou oxyde de propylène étant compris entre 2 et 50 et le nombre de groupements glycérol étant compris entre 2 et 30.

On peut également citer les copolymères d'oxydes d'éthylène et de propylène; les condensats d'oxydes d'éthylène et de propylène sur des alcools gras; les amides gras polyéthoxylés ayant de préférence 2 à 30 moles d'oxyde d'éthylène; les amides gras polyglycérolés comportant 1 à 5 groupements glycérol et en particulier 1,5 à 4; les amines grasses polyéthoxylées ayant de préférence 2 à 30 moles d'oxyde d'éthylène; les esters d'acides gras du sorbitan oxyéthylénés ayant de 2 à 30 moles d'oxyde d'éthylène; les esters d'acides gras de sucrose, les esters d'acides bras du polyéthylèneglycol, les alkyl(C₈-C₁₈)polyglycosides, les oxydes d'amines tels que les oxydes d'alkyl(C₁₀-C₁₄)amines ou de N-acylamidopropylmorpholine.

Les agents tensio-actifs amphotères ou zwittérioniques préférés sont les dérivés d'amines secondaires ou tertiaires aliphatiques, dans lesquels le radical aliphatique est une chaîne linéaire ou ramifiée comportant 8 à 18 atomes de carbone et qui contient au moins un groupe anionique hydrosolubilisant carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆)bétaïnes ou les alkyl(C₈-C₂₀)amidoalkyl(C₁-C₆)sulfobétaïnes.

Parmi les dérivés d'amines, on peut citer les produits vendus sous la dénomination MIRANOL, tels que décrits dans les brevets US-A-2.528.378 et 2.781.354 et classés dans le dictionnaire CTFA, 3ème édition, 1982, sous la dénomination d'Amphocarboxyglycinates et Amphocarboxypropionates.

Les huiles sont utilisées dans les compositions conformes à l'invention dans des proportions comprises de préférence entre 0,05 et 20% et de préférence entre 0,1 et 10% en poids par rapport au poids total de la composition.

Les agents de mise en suspension définis sous (b) utilisés conformément à l'invention, sont présents dans des proportions suffisantes pour assurer la mise en suspension des huiles synthétiques dans les compositions et de préférence dans des proportions comprises entre 0,1 et 20% en poids par rapport au poids total de la composition et en particulier entre 0,5 et 10%.

Les agents tensio-actifs sont utilisés dans les compositions conformes à l'invention dans des proportions suffisantes pour conférer un caractère détergent à la composition et sont comprises de préférence entre 5 et 50% en poids par rapport au poids total de la composition et en particulier entre 8 et 35%.

Les compositions, selon l'invention, présentent un pH généralement compris entre 2 et 9, et plus particulièrement entre 3 et 8.

Le milieu aqueux des compositions est constitué, soit par de l'eau, soit par un mélange d'eau et de solvants cosmétiquement acceptables choisis parmi les alcools inférieurs, les alkylèneglycols et les éthers de glycol.

Les compositions, selon l'invention, peuvent contenir également des agents régulateurs de viscosité, tels que des électrolytes comme le chlorure de sodium ou le xylènesulfonate de sodium, des hydrotropes, des épaississants comme les dérivés de la cellulose, la gomme de guar, des gommes de guar hydroxypropylées.

Ces agents régulateurs de viscosité sont utilisés dans des proportions allant jusqu'à 10% en poids par rapport au poids total de la composition et de préférence inférieure à 5%.

Les compositions conformes à l'invention peuvent éventuellement contenir en outre d'autres agents, pourvu qu'ils n'altèrent pas la stabilité des compositions, tels que des tensio-actifs cationiques, des polymères ou des protéines quaternisées ou non, ou des huiles, cires, résines ou gomme de silicone.

Les polymères, les tensio-actifs cationiques et les protéines quaternisées ou non, les silicones, sont utilisés dans les compositions cosmétiques ou dermatologiques, selon l'invention, dans des proportions comprises entre 0,05 et 6% et de préférence entre 0,1 et 3% par rapport au poids total de la composition.

Les compositions selon l'invention peuvent également contenir différents adjuvants habituellement utilisés en cosmétique, tels que des parfums, des conservateurs, des séquestrants, des stabilisateurs de mousse, des agents propulseurs, des colorants, des agents acidifiants ou alcalinisants ou d'autres adjuvants selon l'usage envisagé.

Les compositions dermatologiques contiennent en outre une substance active pour le traitement des affections dermatologiques.

Les procédés de lavage et/ou de conditionnement des cheveux ou de la peau consistent à appliquer sur ceux-ci une composition telle que définie ci-dessus, cette application étant suivie d'un rinçage.

Les compositions conformes à l'invention sont également utilisables comme gels douche pour le lavage des cheveux et de la peau, auquel cas ils sont appliqués sur la peau et les cheveux humides et sont rincés après application.

Les exemples qui suivent sont destinés à illustrer l'invention sans pour autant présenter un caractère limitatif.

### EXEMPLE 1

On prépare la composition suivante :

| | |
|---|---|
| - Acide éther carboxylique polyoxyéthyléné de formule RO(C₂H₄O)_{ℓ}CH₂COOH où R = alkyle en C₁₂-C₁₄ et ℓ = 4,5 vendu par la Société CHEM'Y sous la dénomination AKYPO RLM 45, à 90% de matière active (MA) | 8,0 g MA |
| - "Cocoamphocarboxyglycinate" (CTFA, 3ème édition, 1982) vendu sous la dénomination MIRANOL C2M Conc. par la Société MIRANOL en solution aqueuse à 38% de matière active (MA) | 4,0 g MA |
| - Oléate de propylèneglycol oxyéthyléné à 55 moles d'oxyde d'éthylène estérifié par l'acide oléique, vendu sous la dénomination ANTIL 141 LIQUID par la Société GOLDSCHMIDT | 2,0 g |
| - Heptaméthylnonane vendu sous la dénomination ARLAMOL HD par la Société ICI (correspondant à la formule (I) où n=3)) | 1,0 g |
| - Conservateurs, parfum, colorants qs | |
| - Eau | qsp 100,0 g |
| - HCl qs pH 7,2 | |

Cette composition est utilisée comme shampooing pour le lavage des cheveux.

### EXEMPLE 2

On prépare la composition suivante :

| | |
|---|---|
| - Alkylpolyglycoside vendu par la Société HORIZON CHEMICAL sous la dénomination APG 300 en solution à 50% de matière active (MA) | 12,5 g MA |
| - Laurylsulfate d'ammonium | 5,0 g |
| - Alkyl(C₁₃-C₁₅)oxydiéthoxyméthylmonoéthanolamide vendu sous la dénomination AMINOL A15 par la Société CHEM'Y | 3,0 g |
| - Iso-hexadécane (correspondant à la formule (I) où n=3)) vendu sous la dénomination PERMETHYL 101 A par la Société PRESPERSE INC | 3,0 g |
| - Conservateurs, parfum qs | |
| - Eau | qsp 100,0 g |
| - HCl qs pH 6,5 | |

Cette composition est utilisée comme shampooing pour le lavage des cheveux.

### EXEMPLE 3

On prépare la composition suivante :

| | |
|---|---|
| - Acide éther carboxylique polyoxyéthyléné de formule RO(C₂H₄O)_{ℓ}CH₂COOH où R = alkyle en C₁₂-C₁₄ et ℓ = 2,5 vendu sous la dénomination AKYPO RLM 25 par la Société CHEM'Y à 90% de matière active (MA) | 5,0 g MA |
| - Isooctahexacontane (correspondant à la formule (I) où n=16)) vendu sous la dénomination PERMETHYL 104 A par la Société PRESPERSE INC | 0,15 g |
| - Isohexadécane (correspondant à la formule (I) où n=3)) vendu sous la dénomination PERMETHYL 101 A par la Société PRESPERSE INC | 0,2 g |
| - Laurylsulfate d'ammonium | 6,0 g |
| - Laurylsarcosinate de Na vendu sous la dénomination ORAMIX L30 par la Société SEPPIC à 30% de matière active (MA) | 5,0 g MA |
| - Distéarate de PEG 6000 | 2,0 g |
| - Conservateurs, parfum qs | |
| - Eau | qsp 100,0 g |
| - HCl qs pH 5 | |

Cette composition est utilisée comme shampooing pour le lavage des cheveux.

### EXEMPLE 4

On prépare la composition suivante :

| | |
|---|---|
| - Scléroglucane vendu sous la dénomination ACTIGUM CS 11 par la Société SANOFI BIO INDUSTRIES à 90% de MA | 1,0 g MA |
| - Laurylsulfate de triéthanolamine | 10,0 g |
| - Laurylbétaïne vendue sous la dénomination DEHYTON AB 30 par la Société HENKEL en solution aqueuse à 32% de MA | 2,0 g MA |
| - Heptaméthylnonane (correspondant à la formule (I) où n=3)) vendu sous la dénomination ARLAMOL HD par la Société ICI | 1,0 g |
| - Conservateurs, parfum qs | |
| - Eau | qsp 100,0 g |
| - NaOH qs pH 7,1 | |

Cette composition est utilisée comme shampooing pour le lavage des cheveux.

### EXEMPLE 5

On prépare la composition suivante :

| | |
|---|---|
| - Laurylsulfate de triéthanolamine | 15,0 g |
| - Gomme de xanthane vendue sous la dénomination KELTROL T par la Société KELCO | 0,5 g |
| - Chlorure de sodium | 2,0 g |
| - Chlorure de N-cétylpyridinium monohydrate | 1,0 g |
| - Isoeicosane (correspondant à la formule (I) où n=4)) vendu sous la dénomination PERMETHYL 102 A par la Société PRESPERSE INC | 2,5 g |
| - Isooctahexacontane vendu sous la dénomination PERMETHYL 104 A par la Société PRESPERSE INC | 0,7 g |
| - Conservateurs, parfum qs | |
| - Eau | qsp 100,0 g |
| - NaOH qs pH 7 | |

Cette composition est utilisée comme shampooing pour le lavage des cheveux.

### EXEMPLE 6

On prépare la composition suivante :

| | |
|---|---|
| - Lauryléthersulfate de sodium oxyéthyléné à 2,2 moles d'oxyde d'éthylène | 10,0 g |
| - Laurylsulfate d'ammonium | 8,0 g |
| - Diester C₁₆-C₁₈ d'éthylèneglycol (30/70 en poids) | 2,0 g |
| - Monoisopropanolamide d'acide de coprah | 2,2 g |
| - Chlorure de sodium | 2,5 g |
| - Heptaméthylnonane (correspondant à la formule (I) où n=3)) vendu sous la dénomination ARLAMOL HD par la Société ICI | 1,0 g |
| - Chlorure de béhényl triméthyl ammonium | 0,5 g |
| - Conservateur, parfum qs | |
| - Eau | qsp 100,0 g |
| - HCl qs pH 6 | |

Cette composition est utilisée comme shampooing pour le lavage des cheveux.

### EXEMPLE 7

On prépare la composition suivante :

| | |
|---|---|
| - Acide éther carboxylique polyoxyéthyléné de formule RO(C₂H₄O)_{ℓ}CH₂COOH où R = alkyle en C₁₂-C₁₄ et ℓ = 4,5 vendu à 90% de matière active (MA) sous la dénomination RLM 45 par la Société CHEM'Y | 10,0 g MA |
| - "Cocoamphocarboxyglycinate" (CTFA, 3ème édition, 1982) vendu sous la dénomination MIRANOL C2M Conc., par la Société MIRANOL en solution aqueuse à 38% de matière active (MA) | 6,0 g MA |
| - Oxyde de diméthylstéarylamine vendu à 25% de matière active sous la dénomination AMMONYX SO par la Société ONYX | 1,5 g MA |
| - Isoeicosane (correspondant à la formule (I) où n=4)) vendu sous la dénomination PERMETHYL 102 A par la Société PRESPERSE INC | 3,5 g |
| - Conservateurs, parfum qs | |
| - Eau | qsp 100,0 g |
| - HCl qs pH 7 | |

Cette composition est utilisée comme shampooing pour le lavage des cheveux.

### EXEMPLE 8

On prépare la composition suivante :

| | |
|---|---|
| - Lauryléthersulfate de sodium oxyéthyléné à 2,2 moles d'oxyde d'éthylène | 12,0 g |
| - Alpha oléfine sulfonate de sodium | 5,0 g |
| - Monoisopropanolamide de coprah | 2,2 g |
| - Cétostéarylsulfate de sodium (C₁₆-C₁₈ 50/50) | 1,5 g |
| - Chlorure de sodium | 2,0 g |
| - Heptaméthylnonane (correspondant à la formule (I) où n=3)) vendu sous la dénomination ARLAMOL HD par la Société ICI | 0,8 g |
| - Conservateurs, parfum qs | |
| - Eau | qsp 100,0 g |
| - NaOH qs pH 6,5 | |

Cette composition est utilisée comme shampooing pour le lavage des cheveux.

### EXEMPLE 9

On prépare la composition suivante :

| | |
|---|---|
| - Alkyl(C₁₂-C₁₄)polyglycoside (comportant un nombre moyen de glucoside égal à 1,4), vendu sous la dénomination TRITON CG 312 par la Société SEPPIC | 35,0 g MA |
| - Mélange de monoéthanolamide d'acide gras de coprah et de glycérol, vendu sous la dénomination AMINOL CM par la Société FINETEX | 5,0 g |
| - Distéarate de glycol | 5,0 g |
| - Isohexapentacontahectane (composé de formule (II) où m=38)) | 0,5 g |
| - Isododécane (composé de formule (I) où n=2)) | 2,0 g |
| - Conservateurs, parfums, colorants qs | |
| - Eau | qsp 100,0 g |

Le pH est ajusté à 6 par de la triéthanolamine.

Cette composition est utilisée comme gel douche.

### EXEMPLE 10

On prépare la composition suivante :

| | |
|---|---|
| - Alkyl(C₁₂-C₁₄)sulfate de triéthanolamine en solution aqueuse à 40% de MA, vendu sous la dénomination EMPICOL TL 40/FL par la Société MARCHON | 12,0 g MA |
| - Gomme de xanthane vendue sous la dénomination KELTROL T par la Société KELCO | 2,0 g |
| - Distéarate de glycol | 2,5 g |
| - Heptaméthylnonane (composé de formule (I) où n=3)) | 5,0 g |
| - Conservateurs, parfums, colorants qs | |
| - Eau | qsp 100,0 g |

Le pH est ajusté à 7 par la triéthanolamine.

Cette composition est utilisée comme shampooing.

### EXEMPLE 11

On prépare la composition suivante :

| | |
|---|---|
| - Acide alkyl(C₁₂-C₁₄)éther carboxylique à 4,5 moles d'oxyde d'éthylène, vendu sous la dénomination AKYPO RLM 45 par la Société LAMBERT RIVIERE | 10,0 g MA |
| - Mélange de cocoylaminopropylbétaïne et de monolaurate de glycérol en solution aqueuse à 35% de MA, vendu sous la dénomination TEGO BETAINE HS par la Société GOLDSCHMIDT | 5,0 g MA |
| - Alpha-oléfine sulfonate de sodium en C₁₄-C₁₆, vendue sous la dénomination WITCONATE AOS par la Société WITCO | 5,0 g |
| - Oléate de propylèneglycol oxyéthyléné à 55 moles d'oxyde d'éthylène et estérifié par l'acide oléique, vendu sous la dénomination ANTIL 141 LIQUID par la Société GOLDSCHMIDT | 2,0 g |
| - Iso-eicosane (composé de formule (I) où n=4)) | 0,05 g |
| - Conservateurs, parfums, colorants qs | |
| - Eau | qsp 100,0 g |

Le pH est ajusté à 6 par de la triéthanolamine.

Cette composition est utilisée comme gel douche.

### EXEMPLE 12

On prépare la composition suivante :

| | |
|---|---|
| - Lauryléthersulfate de sodium vendu sous la dénomination EMPICOL ESB/3FL par la Société MARCHON | 12,0 g MA |
| - Cocoylbétaïne en solution aqueuse à 32% de MA | 5,0 g MA |
| - Monoisopropanolamide d'acide gras de coprah, vendu sous la dénomination EMPILAN CIS par la Société MARCHON | 4,5 g |
| - Distéarate de glycol | 3,0 g |
| - Isohexadécane (composé de formule (I) où n=3)) | 10,0 g |
| - Conservateurs, parfums, colorants qs | |
| - Eau | qsp 100,0 g |

Le pH est ajusté à 6 par de l'acide chlorhydrique.

Cette composition est utilisée comme shampooing.

## Revendications

1. Composition de lavage des matières kératiniques, en particulier des cheveux et/ou de la peau, caractérisée par le fait qu'elle comprend, dans un milieu aqueux:
A) au moins une huile synthétique choisie parmi :
(i) les isoparaffines répondant à la formule : 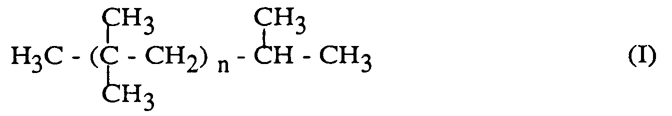 où n est compris entre 2 et 16 inclus;
(ii) le mélange des isoparaffines de formule (I) avec des isoparaffines de formule (II) : 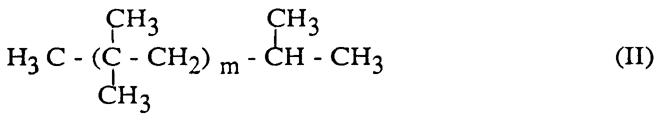 où m est égal ou supérieur à 18, et de préférence compris entre 18 et 40;
la viscosité capillaire des huiles définies sous i) et ii) étant inférieure à 500 cps.
B) un agent de mise en suspension de l'huile, défini sous A), choisi parmi :
i) les composés de formule :
R-X (III)
dans laquelle :
R est un radical aliphatique à longue chaîne carbonée, éventuellement interrompue par un ou plusieurs atomes d'oxygène, et X est un reste acide carboxylique, sulfurique ou phosphorique ou un radical dérivé d'un acide carboxylique ou d'un amide;
(ii) des oxydes d'amines de formule : 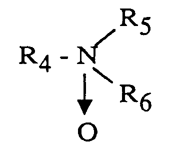 où R₄ est un alkyle en C₁₆-C₂₂, R₅ et R₆, identiques ou différents, représentent un alkyle en C₁-C₄ ou hydroxyalkyle en C₁-C₄;
(iii) les biopolysaccharides;
C) un agent tensio-actif possédant des propriétés détergentes présent dans des proportions suffisantes pour conférer à la composition des propriétés lavantes.

2. Composition selon la revendication 1, caractérisée par le fait que les huiles synthétiques sont choisies parmi les huiles de formule (I), dans lesquelles n est égal à 2,3,4 ou 16, ou les mélanges de ces huiles avec une huile synthétique de formule (II), dans laquelle m est égal à 38.

3. Composition selon la revendication 1, caractérisée par le fait que les composés de formule RX sont choisis parmi ceux dans lesquels :
(i) R est un radical alkyle ou alcényle en C₁₁-C₂₁ et X est
. un groupement COOA où A est un radical mono- ou polyhydroxyalkyle dérivé d'un polyol en C₂-C₃ ou un radical CH₂CH₂SO₃M;
. un groupement CO(OCH₂CH₂)ₖOH où k a une valeur comprise entre 2 et 150;
. un groupement 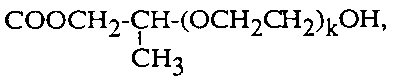 où k a une valeur comprise entre 2 et 150, les fonctions OH libres des groupements définis ci-dessus pouvant être estérifiées par un acide RCOOH où R est un alkyle ou alcényle en C₁₁-C₂₁;
. un groupement CONR₁R₂ où R₁ et R₂ représentent hydrogène ou hydroxyalkyle en C₁-C₄, l'un au moins représentant hydroxyalkyle en C₁-C₄;
. un groupement OSO₃M ou 1/3 PO₄ ³⁻M₃ où M représente un métal alcalin, ammonium ou un reste d'alcanolamine en C₁-C₄;
(ii) R désigne un radical R₃O(C₂H₄O)_{ℓ}CH₂ et X désigne un groupement COOM où M a la signification indiquée ci-dessus, R₃ désignant un radical alkyle en C₁₂-C₁₄ et ℓ un nombre entier ou décimal compris entre 2,5 et 10, ou bien R₃ désigne oléyle et ℓ varie de 2 à 9 ou encore R₃ désigne alkyle en (C₈-C₉) phényle et ℓ varie de 4 à 8, ou les dérivés dans lesquels R₃ désigne un groupement alkyle (C₁₂-C₁₆) et X un groupement CONR₁R₂, dans lequel R₁ et R₂ ont la même signification que celle indiquée ci-dessus et ℓ désigne 1 à 3 inclus.

4. Composition selon l'une quelconque des revendications 1 à 3, caractérisée par le fait que l'on utilise comme agents de mise en suspension des esters d'acides gras en C₁₂-C₂₂ et de polyols en C₂-C₃, choisis parmi les myristates, palmitates et stéarates d'éthylèneglycol, de propylèneglycol ou de glycérol.

5. Composition selon la revendication 4, caractérisée par le fait que les esters d'acides gras et de polyols sont choisis parmi le mono- ou distéarate d'éthylèneglycol ou le mono- ou di- ou tristéarate de glycérol.

6. Composition selon l'une quelconque des revendications 1 à 3, caractérisée par le fait que la composition contient à titre d'agent de mise en suspension, des alcanolamides d'acides gras en C₁₂-C₂₂, choisis parmi le diéthanolamide de coprah ou stéarique, le monoéthanolamide de coprah ou stéarique, le monoisopropanolamide de coprah ou stéarique.

7. Composition selon l'une quelconque des revendications 1 à 3, caractérisée par le fait que l'agent de mise en suspension est un alkyléther d'alcanolamide choisi parmi les alkyl(C₁₃-C₁₅)oxydiéthoxyméthylmonoéthanolamides.

8. Composition selon l'une quelconque des revendications 1 à 3, caractérisée par le fait que les acides gras en C₁₆-C₂₂ polyoxyéthylénés sont choisis parmi les stéarates de polyéthylèneglycol comportant de 4 à 150 motifs éthoxy; que les esters d'acides gras en C₁₆-C₂₂ et de propylèneglycol polyoxyéthylénés et estérifiés sont choisis parmi les composés comportant de 4 à 100 motifs éthoxy.

9. Composition selon l'une des revendications 1 ou 3, caractérisée par le fait que les acides éthers carboxyliques polyoxyéthylénés sont choisis parmi les composés répondant à la formule R₃O(C₂H₄O)_{ℓ} CH₂COOM, dans laquelle R₃ représente une chaîne alkyle en C₁₂-C₁₄ et ℓ désigne 2,5; 3,8; 4,5 ou 10, ou bien dans laquelle R₃ désigne oléyle et ℓ est égal à 5, ou bien des mélanges de composés dans lesquels R₃ désigne oléyle et ℓ a les valeurs 2 et 9, ou encore où R₃ désigne nonylphényle et ℓ a les valeurs 4 et 7, ou bien R₃ représente octylphényle et ℓ a les valeurs 4 et 8.

10. Composition selon l'une des revendications 1 ou 2, caractérisée par le fait que les biopolysaccharides sont choisis parmi les gommes de xanthane ou les scléroglucanes.

11. Composition selon l'une quelconque des revendications 1 à 9, caractérisée par le fait que les agents tensio-actifs détergents sont choisis parmi les agents tensio-actifs anioniques, amphotères, zwittérioniques, non-ioniques ou leurs mélanges.

12. Composition selon la revendication 10, caractérisée par le fait que les agents tensio-actifs anioniques sont choisis parmi les sels alcalins, les sels de magnésium, les sels d'ammonium, les sels d'amines ou les sels d'aminoalcools des composés suivants : les alkyléthersulfates, alkylamidoéthersulfates, alkylarylpolyéthersulfates, monoglycérides sulfates; les alkylsulfonates, alkylamides sulfonates, alkylarylsulfonates, oléfines sulfonates, paraffines sulfonates; les alkylsulfosuccinates, les alkyléthersulfosuccinates, les alkylamides sulfosuccinates, les alkylsulfosuccinamates; les alkylsulfoacétates, les alkylétherphosphates; les acylsarcosinates, N-acyltaurates; le radical alkyle ou acyle de ces différents composés étant constitué par une chaîne carbonée comportant de 12 à 20 atomes de carbone; les alkylsulfates ou alkylphosphates dont le radical alkyle comporte 12 à 14 atomes de carbone; les sels d'acides gras des acides oléique, ricinoléique, palmitique, stéarique; les acides d'huile de coprah ou d'huile de coprah hydrogénée; les acyl lactylates, dont le radical acyle comporte de 8 à 20 atomes de carbone, les acides éthers carboxyliques polyoxyalkylénés.

13. Composition selon la revendication 11, caractérisée par le fait que les agents tensio-actifs non-ioniques sont choisis parmi les alcools ou α-diols ou les alkylphénols et acides gras polyéthoxylés, polyoxypropylénés ou polyglycérolés, à chaîne grasse comportant 8 à 18 atomes de carbone, le nombre de groupements oxyde d'éthylène, oxyde de propylène étant compris entre 2 et 50 et le nombre de groupements glycérol étant compris entre 2 et 30; les copolymères d'oxyde d'éthylène et de propylène; les condensats d'oxyde d'éthylène et de propylène sur des alcools gras; les amides gras polyéthoxylés; les amides gras polyglycérolés; les amines grasses polyéthoxylées; les esters d'acides gras du sorbitan oxyéthylénés; les esters d'acides gras de sucrose ou du polyéthylèneglycol; les alkylpolyglycosides; les oxydes d'alkyl(C₁₀-C₁₄)amines ou de N-acylarnidopropylmorpholine.

14. Composition selon la revendication 11, caractérisée par le fait que les avents tensio-actifs amphotères ou zwittérioniques sont choisis parmi les dérivés d'amines secondes ou tertiaires aliphatiques, dans lesquels le radical aliphatique est une chaîne linéaire ou ramifiée comportant de 8 à 18 atomes de carbone et qui contient au moins un groupe anionique hydrosolubilisant carboxylate, sulfonate, sulfate, phosphate ou phosphonate; les alkyl(C₈-C₂₀)bétaïnes, les sulfobétaïnes, les alkyl(C₈-C₂₀)amidoalkyl(C₁-C₆)bétaïnes ou les alkyl(C₈-C₂₀)amidoalkyl(C₁-C₆)sulfobétaïnes.

15. Composition selon l'une quelconque des revendications 1 à 14, caractérisée par le fait que l'huile est utilisée dans les compositions conformes à l'invention dans des proportions comprises entre 0,05 et 20% et de préférence entre 0,1 et 10% en poids par rapport au poids total de la composition.

16. Composition selon l'une quelconque des revendications 1 à 15, caractérisée par le fait que les agents de mise en suspension définis dans les revendications 1 à 11, sont utilisés dans des proportions suffisantes pour assurer la mise en suspension de l'huile.

17. Composition selon la revendication 16, caractérisée par le fait que les agents de mise en suspension sont présents dans des proportions comprises entre 0,1 et 20% en poids par rapport au poids total de la composition, et en particulier entre 0,5 et 10%.

18. Composition selon l'une quelconque des revendications 1 à 16, caractérisée par le fait que les agents tensio-actifs sont présents dans une proportion suffisante pour conférer un caractère détergent à la composition.

19. Composition selon la revendication 18, caractérisée par le fait que les agents tensio-actifs sont présents dans des proportions comprises entre 5 et 50% en poids par rapport au poids total de la composition et en particulier entre 8 et 35% en poids.

20. Composition selon l'une quelconque des revendications 1 à 19, caractérisée par le fait que le pH est compris entre 2 et 9 et en particulier entre 3 et 8.

21. Composition selon l'une quelconque des revendications 1 à 20, caractérisée par le fait que le milieu aqueux est constitué par de l'eau ou par un mélange d'eau et d'un solvant cosmétiquement acceptable choisi parmi les alcools inférieurs, les alkylèneglycols et les éthers de glycol.

22. Composition selon l'une quelconque des revendications 1 à 21, caractérisée par le fait que la composition contient en plus des agents régulateurs de viscosité choisis parmi les électrolytes, les hydrotropes ou des agents épaississants présents en des proportions pouvant aller jusqu'à 10% en poids par rapport au poids total de la composition.

23. Composition selon l'une quelconque des revendications 1 à 22, caractérisée par le fait qu'elle contient en plus un ou plusieurs adjuvants choisis parmi les tensio-actifs cationiques, les polymères, des protéines éventuellement quaternisées ou une huile, cire, résine ou gomme de silicone.

24. Composition selon l'une quelconque des revendications 1 à 23, caractérisée par le fait qu'elle contient différents adjuvants cosmétiquement acceptables choisis parmi les parfums, les conservateurs, les séquestrants, les synergistes de mousses, les stabilisateurs de mousses, les agents acidifiants ou alcalinisants.

25. Utilisation comme shampooing de la composition telle que définie dans l'une quelconque des revendications 1 à 24.

26. Utilisation comme gel douche de la composition telle que définie dans l'une quelconque des revendications 1 à 24.

27. Procédé de lavage et de conditionnement des matières kératiniques, caractérisé par le fait que l'on applique sur ces matières au moins une composition telle que définie dans l'une quelconque des revendications 1 à 24, et qu'après un temps de pose, on rince à l'eau les matières traitées.

28. Utilisation des composés de formule :
a)
R-X (III)
dans laquelle :
R est un radical aliphatique à longue chaîne carbonée, éventuellement interrompue par un ou plusieurs atomes d'oxygène, et X est un reste acide carboxylique, sulfurique ou phosphorique ou un radical dérivé d'un acide carboxylique ou d'un amide;
b) des oxydes d'amines de formule : 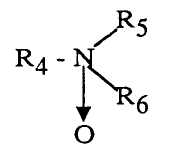 dans laquelle R₄ désigne un groupement alkyle en C₁₆-C₂₂, et R₅ et R₆, identiques ou différents, représentent un groupement alkyle en C₁-C₄ ou hydroxyalkyle en C₁-C₄;
c) les biopolysaccharides;
comme agent de mise en suspension d'huiles synthétiques choisies parmi :
(i) les isoparaffines répondant à la formule : 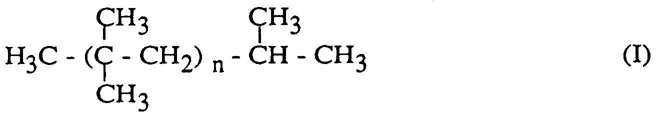 où n est compris entre 2 et 16 inclus;
(ii) le mélange des isoparaffines de formule (I) avec des isoparaffines de formule (II) : 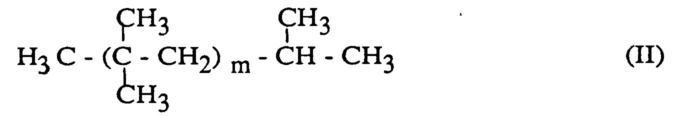 où m est égal ou supérieur à 18, et de préférence compris entre 18 et 40;
dans un milieu aqueux contenant des tensio-actifs détergents.

## Claims

1. Composition for washing keratinous materials, in particular the hair and/or the skin, characterised in that it contains in an aqueous medium:
(A) at least one synthetic oil chosen from:
(i) the isoparaffins of structure: 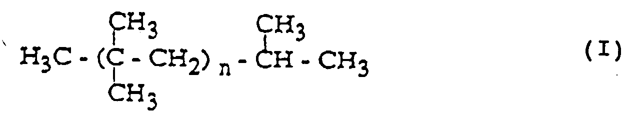 where n is between 2 and 16 inclusive;
(ii) a mixture of the isoparaffins of formula (I) with the isoparaffins of formula (II): 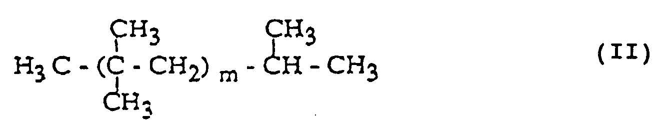 where m is not less than 18, and preferably is between 18 and 40;
the capillary viscosity of the oils defined under i) and ii) being less than 500 cP.s;
(B) an agent for suspending the oil defined under A), chosen from:
i) the compounds of formula:
R-X (III)
in which:
R is a long carbon chain aliphatic radical optionally interrupted by one or more oxygen atoms, and X is a carboxylic, sulphuric or phosphoric acid radical or a radical derived from a carboxylic acid or an amide;
(ii) amine oxides of formula: 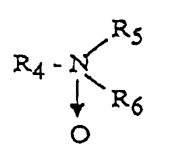 where R₄ is a C₁₆-C₂₂ alkyl, R₅ and R₆, which are identical or different, represent a C₁-C₄ alkyl or C₁-C₄ hydroxyalkyl;
(iii) biopolysaccharides;
C) a surface-active agent possessing detergent properties, present in proportions sufficient to confer washing properties on the composition.

2. Composition according to Claim 1, characterised in that the synthetic oils are chosen from the oils of formula (I), in which n is equal to 2, 3, 4 or 16, or from mixtures of these oils with a synthetic oil of formula (II) in which m is equal to 38.

3. Composition according to Claim 1, characterised in that the compounds of formula RX are chosen from those in which:
(i) R is a C₁₁-C₂₁ alkyl or alkenyl radical and X is
• a COOA group where A is a mono- or polyhydroxyalkyl radical derived from a C₂-C₃ polyol or a radical CH₂CH₂SO₃M;
• a group CO(OCH₂CH₂)ₖOH where k has a value of between 2 and 150;
• a group 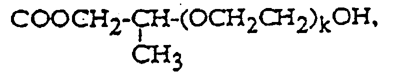 where k has a value of between 2 and 150, it being possible for the free OH functional groups of the groups defined above to be esterified by an acid RCOOH where R is a C₁₁-C₂₁ alkyl or alkenyl;
• a group CONR₁R₂ where R₁ and R₂ represent hydrogen or C₁-C₄ hydroxyalkyl, at least one representing C₁-C₄ hydroxyalkyl;
• a group OSO₃M or 1/3 PO₄³⁻M₃ where M represents an alkali metal, ammonium or a C₁-C₄ alkanolamine residue;
(ii) R denotes a radical R₃O(C₂H₄O)ₗCH₂ and X denotes a group COOM where M has the meaning given above, R₃ denoting a C₁₂-C₁₄ alkyl radical and l an integer or a decimal between 2.5 and 10, or alternatively R₃ denotes oleyl and l ranges from 2 to 9 or alternatively R₃ denotes (C₈-C₉ alkyl)phenyl and l ranges from 4 to 8, or the derivatives in which R₃ denotes a C₁₂-C₁₆ alkyl group and X a group CONR₁R₂, in which R₁ and R₂ have the same meaning as that given above and l has a value of 1 to 3 inclusive.

4. Composition according to any one of Claims 1 to 3, characterised in that esters of C₁₂-C₂₂ fatty acids and C₂-C₃ polyols chosen from ethylene glycol, propylene glycol or glycerol myristates, palmitates and stearates are used as suspending agents.

5. Composition according to Claim 4, characterised in that the esters of fatty acids and polyols are chosen from ethylene glycol mono- or distearate, or glycerol mono- or di- or tristearate.

6. Composition according to any one of Claims 1 to 3, characterised in that the composition contains by way of suspending agent C₁₂-C₂₂ fatty acid alkanolamides chosen from copra or stearic diethanolamide, copra or stearic monoethanolamide, or copra or stearic monoisopropanolamide.

7. Composition according to any one of Claims 1 to 3, characterised in that the suspending agent is an alkanolamide alkyl ether chosen from (C₁₃-C₁₅ alkyl)oxydiethoxymethylmonoethanolamides.

8. Composition according to any one of Claims 1 to 3, characterised in that the polyoxyethylenated C₁₆-C₂₂ fatty acids are chosen from polyethylene glycol stearates containing 4 to 150 ethoxy units; in that the esters of polyoxyethylenated and esterified C₁₆-C₂₂ fatty acids and propylene glycol are chosen from compounds containing 4 to 100 ethoxy units.

9. Composition according to either of Claims 1 and 3, characterised in that the polyoxyethylenated ether carboxylic acids are chosen from the compounds of formula R₃O(C₂H₄O)ₗCH₂COOM, in which R₃ represents a C₁₂-C₁₄ alkyl chain and l denotes 2.5; 3.8; 4.5 or 10, or alternatively in which R₃ denotes oleyl and l is equal to 5, or alternatively mixtures of compounds in which R₃ denotes oleyl and l has the values 2 and 9, or alternatively where R₃ denotes nonylphenyl and l has the values 4 and 7, or alternatively R₃ represents octylphenyl and l has the values 4 and 8.

10. Composition according to either of Claims 1 and 2, characterised in that the biopolysaccharides are chosen from xanthan gums or scleroglucans.

11. Composition according to any one Claims 1 to 9, characterised in that the detergent surface-active agents are chosen from anionic, amphoteric, zwitterionic or nonionic surface-active agents or mixtures thereof.

12. Composition according to Claim 10, characterised in that the anionic surface-active agents are chosen from the alkali metal salts, the magnesium salts, the ammonium salts, the amine salts or the aminoalcohol salts of the following compounds: alkyl ether sulphates, alkyl amidoether sulphates, alkyl aryl polyether sulphates, monoglyceride sulphates; alkyl sulphonates, alkyl amide sulphonates, alkyl aryl sulphonates, olefin sulphonates, paraffin sulphonates; alkyl sulphosuccinates, alkyl ether sulphosuccinates, alkyl amide sulphosuccinates; alkyl sulphosuccinamates; alkyl sulphoacetates; alkyl ether phosphates; acyl sarcosinates, and N-acyltaurates, the alkyl or acyl radical of these various compounds being composed of a carbon chain containing 12 to 20 carbon atoms; alkyl sulphates and alkyl phosphates whose alkyl radical contains 12 to 14 carbon atoms; fatty acid salts of oleic, ricinoleic, palmitic or stearic acids; copra oil or hydrogenated copra oil acids; acyl lactylates whose acyl radical contains 8 to 20 carbon atoms, polyoxyalkylated ether carboxylic acids.

13. Composition according to Claim 11, characterised in that the nonionic surface-active agents are chosen from alcohols or α-diols or alkylphenols and polyethoxylated, polyoxypropyleneated or polyglycerolated fatty acids, with a fatty chain containing 8 to 18 carbon atoms, the number of ethylene oxide or propylene oxide groups being between 2 and 50 and the number of glycerol groups being between 2 and 30; copolymers of ethylene and propylene oxides; condensates of ethylene and propylene oxides with fatty alcohols; polyethoxylated fatty amides; polyglycerolated fatty amides; polyethoxylated fatty amines; oxyethylenated sorbitan fatty acid esters; sucrose or polyethylene glycol fatty acid esters; alkyl polyglycosides; (C₁₀-C₁₄ alkyl)amine or N-acylamidopropylmorpholine oxides.

14. Composition according to Claim 11, characterised in that the amphoteric or zwitterionic surface-active agents are chosen from the aliphatic secondary or tertiary amine derivatives in which the aliphatic radical is a linear or branched chain containing 8 to 18 carbon atoms and which contains at least one anionic water-solubilising carboxylate, sulphonate, sulphate, phosphate or phosphonate group; (C₈-C₂₀ alkyl)betaines, sulphobetaines, (C₈-C₂₀ alkyl)amido(C₁-C₆ alkyl)betaines or (C₈-C₂₀ alkyl)amido(C₁-C₆ alkyl)sulphobetaines.

15. Composition according to any one of Claims 1 to 14, characterised in that the oil is used in the compositions conforming to the invention in proportions of between 0.05 and 20 %, and preferably between 0.1 and 10 % by weight relative to the total weight of the composition.

16. Composition according to any one of Claims 1 to 15, characterised in that the suspending agents defined in Claims 1 to 11 are used in proportions sufficient to ensure the suspension of the oil.

17. Composition according to Claim 16, characterised in that the suspending agents are present in proportions of between 0.1 and 20 % by weight relative to the total weight of the composition, and in particular between 0.5 and 10 %.

18. Composition according to any one of Claims 1 to 16, characterised in that the surface-active agents are present in a proportion sufficient to confer a detergent character on the composition.

19. Composition according to Claim 18, characterised in that the surface-active agents are present in proportions of between 5 and 50 % by weight relative to the total weight of the composition, and in particular between 8 and 35 % by weight.

20. Composition according to any one of Claims 1 to 19, characterised in that the pH is between 2 and 9 and in particular between 3 and 8.

21. Composition according to any one of Claims 1 to 20, characterised in that the aqueous medium is composed of water or a mixture of water and a cosmetically acceptable solvent chosen from lower alcohols, alkylene glycols and glycol ethers.

22. Composition according to any one of Claims 1 to 21, characterised in that the composition in addition contains viscosity regulating agents chosen from electrolytes, hydrotropic agents or thickening agents present in proportions which may range up to 10 % by weight relative to the total weight of the composition.

23. Composition according to any one of Claims 1 to 22, characterised in that it in addition contains one or more adjuvants chosen from cationic surface-active agents, polymers, optionally quaternised proteins or a silicone oil, wax, resin or gum.

24. Composition according to any one of Claims 1 to 23, characterised in that it contains various cosmetically acceptable adjuvants chosen from perfumes, preservatives, sequestering agents, foam synergists, foam stabilisers, acidifying or alkalinising agents.

25. Use as shampoo of the composition as defined in any one of Claims 1 to 24.

26. Use as shower gel of the composition as defined in any one of Claims 1 to 24.

27. Method of washing and conditioning keratinous materials, characterised in that at least one composition as defined in any one of Claims 1 to 24 is applied to these materials, and in that after allowing it to act for a period of time, the treated materials are rinsed with water.

28. Use of the compounds of the formula:
a)
R-X (III)
in which:
R is a long carbon chain aliphatic radical optionally interrupted by one or more oxygen atoms, and X is a carboxylic, sulphuric or phosphoric acid residue or a radical derived from a carboxylic acid or an amide;
b) amine oxides of the formula: 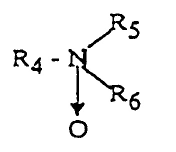 in which R₄ denotes a C₁₆-C₂₂ alkyl group, and R₅ and R₆, which are identical or different, represent a C₁-C₄ alkyl or C₁-C₄ hydroxyalkyl group;
c) biopolysaccharides; as agent for suspending synthetic oils chosen from:
(i) the isoparaffins of formula: 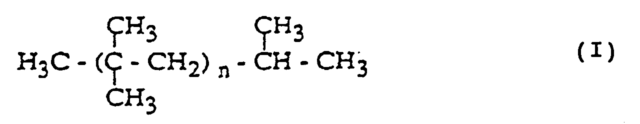 where n is between 2 and 16 inclusive;
(ii) a mixture of the isoparaffins of formula (I) with the isoparaffins of formula (II): 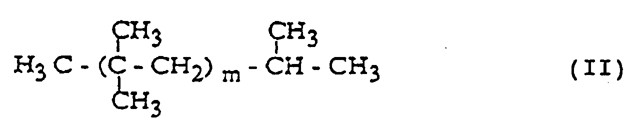 where m is not less than 18, and preferably is between 18 and 40;
in an aqueous medium containing detergent surface-active agents.

## Patentansprüche

1. Zusammensetzung zum Waschen keratinischer Materien, insbesondere der Haare und/oder der Haut, dadurch **gekennzeichnet**, daß sie in einem wässrigen Milieu enthält:
(A) mindestens ein synthetisches Öl, ausgewählt aus:
(i) den Isoparaffinen der Struktur: 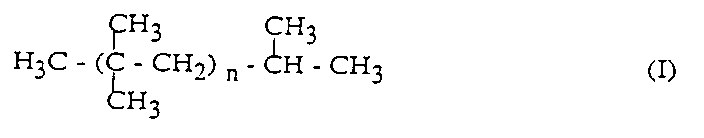 worin n 2 bis 16 beträgt;
(ii) der Mischung der Isoparaffine der Formel (I) mit Isoparaffinen der Formel (II): 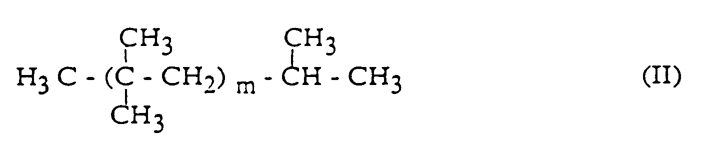 worin m 18 oder mehr und vorzugsweise 18 bis 40 beträgt,
wobei die Kapillarviskosität der unter i) und ii) definierten Öle unterhalb 500 cps liegt;
(B) ein Suspendiermittel für das unter A) definierte Öl, ausgewählt aus:
i) Verbindungen der Formel:
RX (III)
worin gilt:
R ist ein aliphatischer Rest mit langer Kohlenstoffkette, die gegebenenfalls durch ein oder mehrere Sauerstoffatome unterbrochen ist, und X ist ein Carboxyl-, Schwefel- oder Phosphorsäurerest oder ein von einer Carboxylsäure oder einem Amid abgeleiteter Rest;
ii) aus Aminoxiden der Formel: 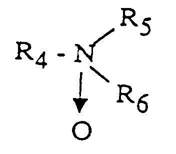 worin R₄ ein C₁₆₋₂₂-Alkylrest ist und R₅ und R₆, gleich oder verschieden, einen C₁₋₄-Alkyl- oder einen Hydroxy-C₁₋₄-alkylrest darstellen;
(iii) aus Biopolysacchariden;
(C) ein oberflächenaktives Mittel mit reinigenden Eigenschaften, das in zur Übertragung von Wascheigenschaften auf die Zusammensetzung ausreichenden Mengen vorhanden ist.

2. Zusammensetzung gemäß Anspruch 1,
dadurch **gekennzeichnet**, daß
die synthetischen Öle aus Ölen der Formel (I), in denen n gleich 2, 3, 4 oder 16 ist, oder aus Mischungen dieser Öle mit einem synthetischen Öl der Formel (II), worin m gleich 38 ist, ausgewählt sind.

3. Zusammensetzung gemäß Anspruch 1,
dadurch **gekennzeichnet**, daß
die Verbindungen der Formel RX aus denjenigen ausgewählt sind, in denen gilt:
(i) R ist ein C₁₁₋₂₁-Alkyl- oder -Alkenylrest,
und X ist
- eine COOA-Gruppe, worin A ein von einem C₂₋₃-Polyol abgeleiteter Mono- oder Polyhydroxyalkylrest oder ein CH₂CH₂SO₃M-Rest ist;
- eine Gruppierung: CO(OCH₂CH₂)ₖOH, worin k einen Wert von 2 bis 150 hat;
- eine Gruppierung: COOCH₂CH-CH₃-(OCH₂CH₂)ₖOH, worin k einen Wert von 2 bis 150 hat, wobei die freien OH-Funktionen der oben definierten Gruppierungen mit einer Säure RCOOH verestert sein können, worin R ein C₁₁₋₂₁-Alkyl oder -Alkenylrest ist;
- eine CONR₁R₂-Gruppe, worin R₁ und R₂ Wasserstoff oder einen Hydroxy-C₁₋₄-alkylrest darstellen, wobei mindestens einer die Hydroxy-C₁₋₄-alkylgruppe darstellt;
- eine OSO₃M- oder 1/3PO₄³⁻M₃-Gruppe, worin M ein Alkalimetall oder den Ammoniumrest oder einen C₁₋₄-Alkanolaminrest darstellt;
(ii) R bedeuten einen R₃O(C₂H₄O)ₗCH₂-Rest und X eine COOM-Gruppe, worin M die oben angegebene Bedeutung hat, wobei R₃ einen C₁₂₋₁₄-Alkylrest und l eine ganze Zahl oder Dezimalzahl von 2,5 bis 10 bedeuten, oder wobei R₃ auch einen Oleylrest bedeutet und l 2 bis 9 beträgt, oder wobei R₃ auch einen C₈₋ ₉-Alkylphenylrest bedeutet und l 4 bis 8 beträgt, oder aus Derivaten, in denen R₃ eine C₁₂₋₁₆-Alkylgruppe und X eine CONR₁R₂-Gruppe bedeuten, in welcher R₁ und R₂ die oben angegebene Bedeutung haben, und in denen l einen Wert von 1 bis 3 aufweist.

4. Zusammensetzung gemäß jedem der Ansprüche 1 bis 3,
dadurch **gekennzeichnet**, daß
man als Suspendiermittel Ester von C₁₂₋₂₂-Fettsäuren und C₂₋ ₃-Polyolen verwendet, die aus den Myristaten, Palmitaten und Stearaten von Ethylenglycol, Propylenglycol oder von Glycerin ausgewählt sind.

5. Zusammensetzung gemäß Anspruch 4,
dadurch **gekennzeichnet**, daß
die Fettsäureester der Polyole aus dem Mono- oder Distearat von Ethylenglycol oder dem Mono- oder Di- oder Tristearat von Glycerin ausgewählt sind.

6. Zusammensetzung gemäß jedem der Ansprüche 1 bis 3,
dadurch **gekennzeichnet**, daß
die Zusammensetzung als Suspendiermittel Alkanolamide von C₁₂₋₂₂-Fettsäuren enthält, die aus dem Diethanolamid von Kopra oder von Stearinsäure, dem Monoethanolamid von Kopra oder von Stearinsäure und dem Monoisopropanolamid von Kopra oder Stearinsäure ausgewählt sind.

7. Zusammensetzung gemäß jedem der Ansprüche 1 bis 3,
dadurch **gekennzeichnet**, daß
das Suspendiermittel ein Alkyletheralkanolamid ist, das aus C₁₃₋₁₅-Alkyloxydiethoxymethylmonoethanolamiden ausgewählt ist.

8. Zusammensetzung gemäß jedem der Ansprüche 1 bis 3,
dadurch **gekennzeichnet**, daß
die polyoxethylierten C₁₆₋₂₂-Fettsäuren aus Stearaten von 4 bis 150 Ethoxy-Einheiten aufweisendem Polyethylenglycol und die polyoxethylierten und veresterten Ester von C₁₆₋₂₂-Fettsäuren und Propylenglycol aus Verbindungen ausgewählt sind, die 4 bis 100 Ethoxy-Einheiten aufweisen.

9. Zusammensetzung gemäß jedem der Ansprüche 1 bis 3,
dadurch **gekennzeichnet**, daß
die polyoxethylierten Ethercarboxylsäuren aus Verbindungen der Formel R₃O(C₂H₄O)ₗCH₂COOM ausgewählt sind, in welcher R₃ eine C₁₂₋₁₄-Alkylkette darstellt und l 2,5; 3,8; 4,5 oder 10 bedeutet, oder in welcher R₃ auch einen Oleylrest bedeutet und l 5 ist, oder auch aus Mischungen von Verbindungen ausgewählt sind, in welchen R₃ einen Oleylrest bedeutet und l die Werte 2 und 9 hat, oder worin R₃ auch einen Nonylphenylrest bedeutet und l die Werte 4 und 7 hat, oder R₃ auch einen Octylphenylrest darstellt und l die Werte 4 und 8 hat.

10. Zusammensetzung gemäß einem der Ansprüche 1 oder 2,
dadurch **gekennzeichnet**, daß
die Biopolysaccharide aus Xanthan-Gummiprodukten oder Scleroglucanen ausgewählt sind.

11. Zusammensetzung gemäß jedem der Ansprüche 1 bis 9,
dadurch **gekennzeichnet**, daß
die oberflächenaktiven reinigenden Mittel aus oberflächenaktiven anionischen, amphoteren, zwitterionischen oder nicht-ionischen Mitteln oder aus deren Mischungen ausgewählt sind.

12. Zusammensetzung gemäß Anspruch 10,
dadurch **gekennzeichnet**, daß
die anionischen oberflächenaktiven Mittel aus den Alkali-, Magnesium-, Ammonium-, Amin- oder Aminoalkoholsalzen der folgenden Verbindungen ausgewählt sind: von Alkylethersulfaten, Alkylamidoethersulfaten, Alkylarylpolyethersulfaten, Monoglycerylsulfaten, Alkylsulfonaten, Alkylamidsulfonaten, Alkylarylsulfonaten, Olefinsulfonaten, Paraffinsulfonaten, Alkylsulfosuccinaten, Alkylethersulfosuccinaten, Alkylamidsulfosuccinaten, Alkylsulfosuccinamaten, Alkylsulfoacetate, Alkyletherphosphaten, Acylsarcosinaten und von N-Acyltauraten, wobei der Alkyl- oder Acylrest dieser verschiedenen Verbindungen aus einer Kohlenstoffkette mit 12 bis 20 Kohlenstoffatomen aufgebaut ist, aus Alkylsulfaten oder Alkylphosphaten, deren Alkylrest 12 bis 14 Kohlenstoffatome aufweist; aus Salzen von Fettsäuren der Öl-, Rizinolen-, Palmitin- oder Stearinsäuren; aus Säuren vom Öl von Kopra oder dem hydrierten Öl von Kopra, aus Acyllactylaten, deren Acylrest 8 bis 20 Kohlenstoffatome aufweist, sowie aus polyoxalkylierten Ethercarboxylsäuren.

13. Zusammensetzung gemäß Anspruch 11,
dadurch **gekennzeichnet**, daß
die nicht-ionischen oberflächenaktiven Mittel aus polyethoxylierten, polyoxpropylierten oder polyglycerierten Alkoholen oder α-Diolen oder Alkylphenolen oder Fettsäuren, mit jeweils einer Fettkette, die 8 bis 18 Kohlenstoffatome aufweist, wobei die Anzahl der Gruppierungen von Ethylenoxid und Propylenoxid 2 bis 50 und die Anzahl der Glycerylgruppierungen 2 bis 30 betragen, aus Copolymeren von Ethylen- und Propylenoxid, aus Kondenatoen von Ethylen- und Propylenoxid an Fettalkohole, aus polyethoxylierten Fettamiden, polyglycerierten Fettamiden, polyethoxylierten Fettaminen, oxethylierten Sorbitanfettsäuren, aus Fettsäureestern von Suchrose oder von Polyethylenglycol, aus Alkylpolyglycosiden, aus C₁₀₋₁₄-Alkylaminoxiden oder aus N-Acylamidopropylmorpholin ausgewählt sind.

14. Zusammensetzung gemäß Anspruch 11,
dadurch **gekennzeichnet**, daß
die amphoteren oder zwitterionischen oberflächenaktiven Mittel aus Derivaten sekundärer oder tertiärer aliphatischer Amine, in denen der aliphatische Rest eine lineare oder verzweigte Kette mit 8 bis 18 Kohlenstoffatomen ist, und mindestens eine anionische, Wasserlöslichkeit vermittelnde Carboxylat-, Sulfonat-, Sulfat-, Phosphat- oder Phosphonatgruppe enthält, aus C₈₋₂₀-Alkylbetainen, Sulfobetainen, C₈₋₂₀-Alkylamido-C₁₋₆-alkylbetainen oder aus C₈₋₂₀-Alkylamido-C₁₋₆-alkylsulfobetainen ausgewählt sind.

15. Zusammensetzung gemäß jedem der Ansprüche 1 bis 14,
dadurch **gekennzeichnet**, daß
das Öl in den erfindungsgemäßen Zusammenetzungen in Mengenanteilen von 0,05 bis 20 und vorzugsweise von 0,1 bis 10 Gew.% verwendet wird, bezogen auf das Gesamtgewicht der Zusammensetzung.

16. Zusammensetzung gemäß jedem der Ansprüche 1 bis 15,
dadurch **gekennzeichnet**, daß
die in den Ansprüchen 1 bis 11 definierten Suspendiermittel in Mengenanteilen eingesetzt sind, die ausreichen, um die Suspendierung des Öls zu gewährleisten.

17. Zusammensetzung gemäß Anspruch 16,
dadurch **gekennzeichnet**, daß
die Suspendiermittel in Mengenanteilen von 0,1 bis 20 und insbesondere von 0,5 bis 10 Gew.% vorhanden sind, bezogen auf das Gesamtgewicht der Zusammensetzung.

18. Zusammensetzung gemäß jedem der Ansprüche 1 bis 16,
dadurch **gekennzeichnet**, daß
die oberflächenaktiven Mittel in einer Menge vorhanden sind, die ausreicht, um auf die Zusammensetzung einen reinigenden Charakter zu übertragen.

19. Zusammensetzung gemäß Anspruch 18,
dadurch **gekennzeichnet**, daß
die oberflächenaktiven Mittel in Mengenanteilen von 5 bis 50 und insbesondere von 8 bis 35 Gew.% vorhanden sind, bezogen auf das Gesamtgewicht der Zusammensetzung.

20. Zusammensetzung gemäß jedem der Ansprüche 1 bis 19,
dadurch **gekennzeichnet**, daß
der pH-Wert 2 bis 9 und insbesondere 3 bis 8 beträgt.

21. Zusammensetzung gemäß jedem der Ansprüche 1 bis 20,
dadurch **gekennzeichnet**, daß
das wässrige Milieu aus Wasser oder einer Mischung aus Wasser und einem kosmetisch geeigneten Lösungsmittel zusammengesetzt ist, das aus Niedrigalkoholen, Alkylenglycolen und aus Glycolethern ausgewählt ist.

22. Zusammensetzung gemäß jedem der Ansprüche 1 bis 21,
dadurch **gekennzeichnet**, daß
die Zusammensetzung zusätzlich Viskositätsreguliermittel enthält, die aus Elektrolyten, Hydrotropen oder aus Verdickungsmitteln ausgewählt sind, wobei diese Mittel in Mengenanteilen vorhanden sind, die bis zu 10 Gew.% ausmachen können, bezogen auf das Gesamtgewicht der Zusammensetzung.

23. Zusammensetzung gemäß jedem der Ansprüche 1 bis 22,
dadurch **gekennzeichnet**, daß
sie zusätzlich einen oder mehrere Hilfsstoffe enthält, die aus kationischen oberflächenaktiven Mitteln, Polymeren, gegebenenfalls quaternierten Proteinen oder aus einem Öl, Wachs, Harz oder einem Silikon-Gummi ausgewählt sind.

24. Zusammensetzung gemäß jedem der Ansprüche 1 bis 23,
dadurch **gekennzeichnet**, daß
sie verschiedene weitere, kosmetisch geeignete Hilfsstoffe enthält, die aus Parfüm-Produkten, Konservierungsstoffen, Sequestriermitteln, Schaumsynergisten, Schaumstabilisatoren, sauer oder alkalisch stellenden Mitteln ausgewählt sind.

25. Verwendung der in einem jeden der Ansprüche 1 bis 24 definierten Zusammensetzung als Shampoo.

26. Verwendung der in einem jeden der Ansprüche 1 bis 24 definierten Zusammensetzung als Duschgel.

27. Verfahren zum Waschen und Konditionieren keratinischer Materien,
dadurch **gekennzeichnet**, daß
man auf diese Materien mindestens eine in jedem der Ansprüche 1 bis 24 definierte Zusammensetzung aufbringt und nach einer Verweilzeit die behandelten Materien mit Wasser spült.

28. Verwendung der Verbindungen der Formel:
a)
R-X (III)
worin gilt:
R ist ein aliphatischer Rest mit langer Kohlenstoffkette, die gegebenenfalls durch ein oder mehrere Sauerstoffatome unterbrochen ist, und X ist ein Carboxyl-, Schwefel- oder Phosphorsäurerest oder ein von einer Carboxylsäure oder einem Amid abgeleiteter Rest;
b) von Aminoxiden der Formel: 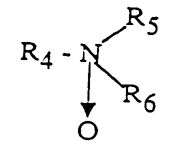 worin R₄ ein C₁₆₋₂₂-Alkylrest ist und R₅ und R₆, gleich oder verschieden, einen C₁₋₄-Alkyl- oder einen Hydroxy-C₁₋₄-alkylrest darstellen;
c) von Biopolysacchariden
als Suspendiermittel von synthetischen Ölen, die ausgewählt sind aus:
(i) den Isoparaffinen der Struktur: 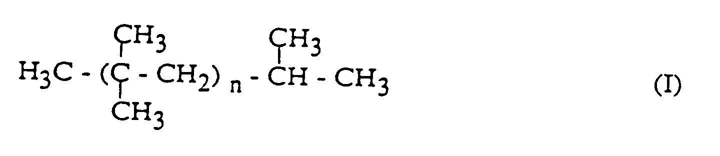 worin n 2 bis 16 beträgt;
(ii) der Mischung der Isoparaffine der Formel (I) mit Isoparaffinen der Formel (II): 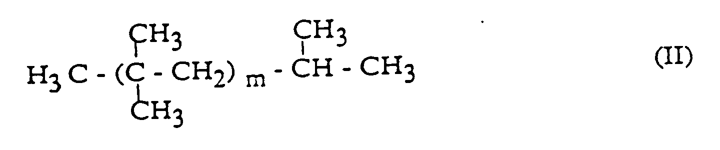 worin m 18 oder mehr und vorzugsweise 18 bis 40 beträgt,
in einem wässrigen Milieu, das oberflächenaktive Detergentien enthält.
